# EUROPEAN PATENT SPECIFICATION

(11) **EP 3 684 393 B1**
(45) Date of publication and mention of the grant of the patent: **27.05.2026**
(21) Application number: 18773865.3
(22) Date of filing: 17.09.2018
(51) Int. Cl.: A61K 36/752, A61K 36/60

(54) **METHOD FOR PREPARING A BOTANICAL EXTRACT OF ABSCISIC ACID**
VERFAHREN ZUR HERSTELLUNG VON ABSCISINSÄURE AUS PFLANZLICHEM EXTRAKT
PROCÉDÉ DE PRÉPARATION D'UN ACIDE ABSCISIQUE D'EXTRAIT BOTANIQUE

(30) Priority: 18.09.2017 EP 17382616
(43) Date of publication of application: 29.07.2020
(73) Proprietor: Euromed, S.A., 08100 Mollet Del Vallès (Barcelona) (ES)
(72) Inventor: RULL PROUS, Santiago, 08034 Barcelona (ES); MULA DALTELL, Anna, 08041 Barcelona (ES); ROIG ALMIRALL, Francisco Javier, 08021 Barcelona (ES); VILLAR GONZALEZ, Agustin, 08291 Ripollet (Barcelona) (ES)
(74) Representative: Durán-Corretjer, S.L.P.
(86) International application number: PCT/IB2018/057113
(87) International publication number: WO 2019/053673

(56) References cited:
- WO-A1-2016/009399
- LOVEYS ET AL.: "Improved extraction of abscisic acid from plant tissue", AUS. J. PLANT PHYSIOL., vol. 15, 1988, pages 421 - 427, XP009503644
- MIRKO MAGNONE ET AL: "Microgram amounts of abscisic acid in fruit extracts improve glucose tolerance and reduce insulinemia in rats and in humans", THE FASEB JOURNAL, vol. 29, no. 12, 1 December 2015 (2015-12-01), US, pages 4783 - 4793, XP055452943, ISSN: 0892-6638, DOI: 10.1096/fj.15-277731
- TURECKOVA V ET AL: "Profiling ABA metabolites in Nicotiana tabacum L. leaves by ultra-performance liquid chromatography-electrospray tandem mass spectrometry", TALANTA, ELSEVIER, AMSTERDAM, NL, vol. 80, no. 1, 15 November 2009 (2009-11-15), pages 390 - 399, XP026641631, ISSN: 0039-9140, [retrieved on 20090617], DOI: 10.1016/J.TALANTA.2009.06.027
- ZHOU R ET AL: "Rapid extraction of abscisic acid and its metabolites for liquid chromatography-tandem mass spectrometry", JOURNAL OF CHROMATOGRAP, ELSEVIER, AMSTERDAM, NL, vol. 1010, no. 1, 22 August 2003 (2003-08-22), pages 75 - 85, XP004446420, ISSN: 0021-9673, DOI: 10.1016/S0021-9673(03)01029-X
- CORRADI M G ET AL: "QUANTITATIVE ANALYSIS OF ABSCISIC-ACID BY HIGH PRESSURE LIQUID CHROMATOGRAPHY IN FRUITS OF PRUNUS-CERASIFERA-VAR-ATROPURPUREA", ACTA NATURALIA DE L'ATENEO PARMENSE1983,, vol. 19, no. 3, 1 January 1983 (1983-01-01), pages 83 - 86, XP009503643, ISSN: 0004-654X

## Description

### Technical field

The present invention relates to a method for preparing an extract of abscisic acid from dried figs which can be used in pharmaceutical, nutritional and cosmetic compositions.

### Prior art

Abscisic acid (hereinafter ABA) is a plant hormone which was discovered at the beginning of the 1960s. Said acid regulates important functions thereof, such as the response to water and nutrient scarcity, ultraviolet radiation, seed dormancy and germination, and root growth.

Natural ABA consists of a mixture of two isomers, which have the following structures:

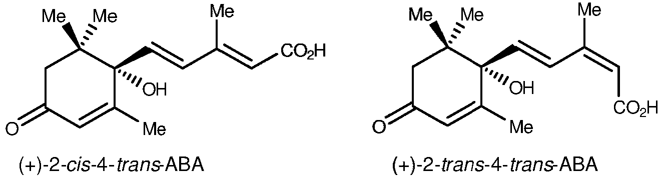

ABA is synthesised in leaves, stems, roots and green fruits. However, it was not until the discovery of the biosynthesis thereof in animal cells that interest has been raised in elucidating possible parallels between its role in plant systems and animals.

Thus, according to the article by Bassaganya-Riera et al., Abscisic acid regulates inflammation via ligand-binding domain-independent activation of peroxisome proliferator-activated receptor, J. Biol. Chem., 2011, 286(4), 2504-2516, ABA received attention as a result of its medical applications. Said article describes how the oral administration of ABA has been shown to be prophylactically and therapeutically effective in murine models of diabetes, inflammatory intestinal complaints and atherosclerosis.

The article by Bassaganya-Riera et al., Mechanisms of action and medicinal applications of abscisic acid, Curr. Med. Chem., 2010, 17(5), 467-478, describes how the use of ABA has been claimed in cancer therapy. Said use is also disclosed in Chinese patent application CN-A-1748674, and in US patent US-2006/0292215.

International patent application WO-A-2007/092556 describes the use of ABA in the treatment of a subject who has or has a predisposition to develop a disease or disorder such as insulin resistance, hyperglycaemia, glucose intolerance, high concentrations of glucose when fasting, type 2 diabetes, prediabetes or inflammation, among others.

The international patent application WO-A-2011/150160 describes the use of ABA for treating or preventing inflammation produced by exposure to lipopolysaccharides or respiratory inflammation.

The international patent application WO-A-2016/009399 describes the use of ABA for the preventive, control or therapeutic treatment of hyperglycaemia with no substantial increase in insulinaemia.

The discovery of ABA and the possible use thereof in medicine has favoured the development of various synthesis routes, as described, for example, in Constantino et al., A novel synthesis of (+-)-abscisic acid, J. Org. Chem., 1989, 54(3), 681-683, or in Smith et al., Concise enantioselective synthesis of abscisic acid and a new analogue, Org. Biomol. Chem., 2006, 4(22), 4186-4192.

In the prior art, industrial methods for obtaining ABA by fermentation from microorganisms have been also proposed, such as those described, for example, in Chinese patent applications CN-1749401, CN-A-1944385, CN-A-101041837 or CN-A-105541603, among others. Said methods have drawbacks, such as, for example, the use of large installations owing to the volumes used in the fermentation, the sensitivity of the microorganisms to contamination or inactivation. The products obtained in this way are practically equivalent to products obtained synthetically, except that this concerns a product obtained by extraction from plant material.

The interest in ABA has also required the development of analysis methods for the quantification thereof, whether in fermentation starter mediums or in samples of plant material.

Thus, for example, Chinese patent application CN-103529156 describes a method for extracting and determining ABA in strawberries, comprising extraction from frozen, ground strawberry pulp using a mixture of methanol, ethyl acetate and formic acid in a proportion of 50:50:1, filtration, evaporation of the sample and UHPLC analysis.

In the article by Loveys et al., Improved extraction of abscisic acid from plant tissue, Aus. J. Plant Physiol., 1988, 15, 421-427, a method is described for determining ABA in small samples of plant tissue with a weight of between 0.7 and 1.0 g. Said method uses methanol, cold water or warm water as a solvent for extracting ABA from the leaves of plants such as apricot trees, lupins, avocado pears, sunflowers and vines. The same article also describes how mainly methanol was used in the past as a solvent to produce quantitative estimates of ABA in said tissues, although alternatives had also been suggested such as the use of a combination of chloroform and methanol; ethanol; ethyl ether; and a combination of methanol, ethyl acetate and acetic acid.

Other articles on analytical methods to determine ABA in plant samples which include a laboratory-scale extraction method are, for example, Cai et al., Sequential solvent induced phase transition extraction for profiling of endogenous phytohormones in plants by liquid chromatography-mass spectrometry, J. Chromat. B: Anal. Technol. Biomed. Life Sci., 2015, 1004, 23-29; Cai et al., Profiling of phytohormones in rice under elevated cadmium concentration, J. Chromat. A, 2015, 1406, 78-86; or Zhang et al., Analysis of phytohormones in vermicompost using a novel combinative sample preparation strategy of ultrasound-assisted extraction and solid-phase extraction coupled with liquid chromatography-tandem mass spectrometry levels by magnetic solid-phase extraction coupled with liquid chromatography tandem mass spectrometry, Talanta, 2015, 139, 189-197.

The aforementioned international patent application WO-A-2016/009399 describes a method for extracting ABA in relatively small quantities from plant matrices (350 g) that have previously undergone a drying process, which entails extraction using carbon dioxide in supercritical conditions, followed by extraction using water. Also described is the extraction using water from dry plant matrices which also includes the ABA and glucose ester (ABB-GE). As described in Lee et al., Activation of Glucosidase via Stress Induced Polymerization Rapidly Increases Active Pools of Abscisic Acid, Cell, 2006, 126, 1109-1120, this is an inactive ABA conjugate which can be transformed into ABA by alkaline hydrolysis, for example. Said patent application describes the ABA content in fruits and vegetables that are common in the western diet. Thus, 2812.3 pmol/g is found in figs, 2583.4 pmol/g in apricots, 1444.2 pmol/g in cranberries/blueberries, 835.4 pmol/g in bananas, 119.2 pmol/g in potatoes, 117.4 pmol/g in soya milk, 90,2 pmol/g in apples (Smith variety) and 52 pmol/g in olives. Magnone et al., 2015, The Faseb Journal, 29, 4783-4793, discloses a process for the extraction of ABA from undried fruits such as figs.

Therefore, the industrial preparation of extracts of abscisic acid has proved difficult in practice owing to the low content thereof in plant materials, which creates difficulties for carrying out economical extraction and marketing the products under economically acceptable conditions. It is therefore not surprising that no methods for extracting ABA from plant materials have been described which are suitable for obtaining extracts rich in ABA on an industrial scale and are suitable for incorporation in pharmaceutical, nutritional or cosmetic compositions.

Accordingly, there is still a need for a method for extracting abscisic acid from plant materials which provides good yields and is easily applied on an industrial scale.

### Subject matter of the invention

The present invention relates to a method for preparing a botanical extract of abscisic acid from dried figs, comprising:
a) extracting a plant material which contains abscisic acid using an aqueous solvent, and separating the solid plant material from the liquid fraction,
b) concentrating the liquid fraction,
c) purifying the liquid fraction obtained at step b) by chromatographic separation in an adsorption column using an organic solvent miscible in water as an eluent, or liquid-liquid extraction using a solvent that is not miscible in water, and
d) evaporating the organic phase obtained at step c);

wherein in step a) said aqueous solvent is water at a temperature of between 50 ºC to 75ºC; and
wherein said botanical extract of abscisic acid comprises between 1000 ppm and 4000 ppm abscisic acid.

### Detailed description of the invention

The present invention relates to a method for preparing a botanical extract of abscisic acid from dried figs, which comprises:
a) extracting a plant material which contains abscisic acid using an aqueous solvent, and separating the solid plant material from the liquid fraction,
b) concentrating the liquid fraction,
c) purifying the liquid fraction obtained at step b) by means of chromatographic separation in an adsorption column using an organic solvent miscible in water as an eluent, or liquid-liquid extraction using a solvent that is not miscible in water, and
d) evaporating the organic phase obtained at step c);

wherein in step a) said aqueous solvent is water at a temperature of between 50 ºC to 75ºC; and
wherein said botanical extract of abscisic acid comprises between 1000 ppm and 4000 ppm abscisic acid.

The authors of the present invention have developed a method for extracting ABA from plant materials which, surprisingly, allows purified extracts having an ABA content of between 1000 ppm and 4000 ppm to be obtained. The method developed allows a plant extract having these high ABA contents to be prepared on an industrial scale starting from raw materials in which the natural content of this product is very small, at levels of approximately 5 ppm, for dry products having a water content of approximately 10 wt.%, and approximately ten times less ABA in undried products. The method developed includes extraction and purification processes in which no chemical synthesis or modification process is carried out. An additional advantage of the process developed is the efficiency thereof given that an ABA recovery of over 80% with respect to the quantity thereof in the initial plant substances is usually obtained. Said method can be applied to a wide range of plant materials, can easily scaled up to an industrial scale and is economically viable for the preparation of extracts suitable for being formulated as pharmaceutical, nutritional or cosmetic compositions.

Throughout the present description, unless otherwise specified, the concentrations expressed as percentages always refer to the percentage by weight (wt.%), that is, grams of a particular ingredient per 100 g of composition, and the percentages of the various ingredients of a composition are adjusted so that the sum thereof is 100%.

In the present description, and also in the claims, the singular forms 'a' and 'the' include the plural reference unless the context clearly indicates otherwise. The ranges defined by the preposition 'between' include the extremes thereof.

### Plant material

The plant material used in the method according to the invention is selected from among those materials that comprise ABA among their ingredients, either in free form or in the form of an ester, such as, for example, the ester with glucose. These plant materials are selected from dried figs. The dry plant material is used, usually with a water content of no more than 20 wt.%, preferably no more than 15 wt.%, and still more preferably no more than 10 wt.%.

In the extraction method, said plant material may be used 'as is', i.e. whole, or mechanically chopped as a stage prior to extraction. In this case, any of the methods available to the person skilled in the art for shredding or cutting up plant material is suitable.

### Solvent

The solvent used in the method according to the invention is an aqueous solvent, that is selected from water.

Extraction is carried out using water as the only solvent.

A volume of solvent equivalent to between 3 and 6 times the weight of the plant material to be extracted is generally used.

### Extraction, filtration and concentration

The extraction method may be carried out in accordance with methods that are well known to the person skilled in the art. That is, by subjecting the plant material, which is whole or cut into small chunks, preferably with a size of less than 2 cm, to the action of water.

To carry out the extraction, conventional solid-liquid extraction methods are suitable, such as for example, maceration, digestion, maceration with stirring, fluidised bed extraction, ultrasound extraction, counter-current extraction, percolation, re-percolation, low-pressure extraction or solid-liquid extraction with continuous reflux.

On an industrial scale, the preferred method is percolation with solvent recycling, also known as repercolation. In said method, the solvent is passed through the plant material continuously, until the extraction is complete. Generally, percolation includes a prior step which consists of wetting the plant material outside the percolator. This operation facilitates contact of the solvent with the plant material to be extracted, increases the porosity of the cell wall and facilitates the diffusion of the extractable substances to the outside of the cells.

In the method according to the invention, extraction is carried out at a temperature of between 50ºC and 75ºC, preferably between 55ºC and 70ºC, and more preferably between 60ºC and 65ºC.

The time needed for the extraction is usually adapted to the initial plant material, the extraction method, the extraction temperature, the ratio between solvent and plant material and similar.

After extraction, the micelle (mixture of solvent and extracted compounds which is formed after the solvent comes in contact with the plant material and has begun to extract the soluble compounds) is usually separated by solid-liquid filtration of the plant material by methods that are well established in industrial processes.

At industrial scale, where a large volume of extract is obtained and sugar concentration in said extract is high, it makes said extract very sensitive to microbial degradation. To avoid this, it is preferably to pasteurize the extract obtained in order to keep its microbiological integrity during the concentration and purification steps.

The liquid fraction obtained after separation of the plant material - the micelle - is concentrated, preferably under vacuum, for example at a pressure of approximately 80 mbar and, generally, at a temperature of no more than 70ºC, preferably no more than 65ºC. Said concentration process is usually continued until a product is obtained having a dry residue that is suitable for the subsequent purification steps, which is preferably between 5 wt.% and 20 wt.%, more preferably between 7 wt.% and 15 wt.%, and still more preferably approximately 10 wt.%.

Optionally, the micelle may undergo a decolouration step to eliminate the colour thereof, for example by treatment with activated carbon or bleaching earths.

In the extraction method according to the invention, extracts are usually obtained which, once dried under vacuum and, generally, at a temperature of no more than 70ºC, preferably no more than 65ºC, contain between 5 and 25 ppm ABA, preferably between 10 ppm and 20 ppm, generally with a (DER) (ratio of the initial plant material to the extract obtained) of between 2 and 3.

### Purification

The method according to the invention comprises a step of purifying the product obtained after extraction using the aqueous solvent.

It has been noted, surprisingly, that the use of: i) chromatographic separation in a chromatographic adsorption column using an organic solvent miscible in water, for example ethanol, as an eluent, or ii) liquid-liquid extraction using a solvent that is not miscible in water, such as, for example, ethyl acetate, allowed extracts having an ABA content of between 1000 and 4000 ppm to be obtained, characterised by a DER of between 200:1 and 1000:1.

Preferably the eluent is selected from among a lower aliphatic alcohol having 1 to 4 carbon atoms, ketone, dioxane, tetrahydrofuran, acetonitrile and mixtures thereof; more preferably the solvent miscible in water is a lower aliphatic alcohol having 1 to 4 carbon atoms, preferably selected from among methanol, ethanol and isopropanol; more preferably the lower aliphatic alcohol is ethanol.

For chromatographic separation, methods are followed that are well known to the person skilled in the art. In said operation, the liquid fraction obtained after extraction using the aqueous solvent is used, filtered to separate the plant material, and concentrated to obtain a solution having a dry residue suitable for being loaded in a chromatographic column filled with an adsorption resin, and an organic solvent miscible in water is used as an eluent, preferably a lower aliphatic alcohol having 1 to 4 carbon atoms; more preferably the eluent is selected from among the group consisting of methanol and ethanol, and still more preferably it is ethanol.

Suitable resins for adsorption chromatography are well known on the market. Among the suitable adsorption resins for carrying out said purification are, for example, resins of the series Amberlite^{®} (Dow), Resindion^{®} (Mitsubishi), or Macronet^{®} (Purolite).

In liquid-liquid extraction, the liquid fraction obtained after extraction using the aqueous solvent is also used, filtered to separate the plant material, and concentrated to obtain a solution having a suitable dry residue, and an organic solvent that is not miscible in water, such as butanol or ethyl acetate, preferably ethyl acetate, is also used. Said extraction is usually carried out at a minimum temperature of 25ºC, the upper limit being the boiling temperature of the organic solvent. Preferably the liquid-liquid extraction is carried out at between 40ºC and 60ºC, more preferably between 45ºC and 55ºC, and still more preferably at 50ºC.

### Extract

The disclosure relates to the extract that can be obtained in accordance with the method according to the invention.

The purified extract of the invention, in solid form, has an ABA content of between 1000 ppm and 4000 ppm, characterised by a DER (ratio between the initial plant material and the extract obtained) of between 200:1 and 1000:1. This level of purification of the extract obtained and the DER thereof shows the great efficiency of said process. This is demonstrated by the fact that the dry plant material used as the raw material in the method according to the invention has an ABA content of approximately 5 ppm and the purifications carried out concentrate this value to levels of between 1000 ppm and 4000 ppm, which, combined with a DER of between 200:1 and 1000:1, represents total yields of at least 80% in terms of ABA recovery.

Accordingly, the disclosure relates to a purified botanical extract of abscisic acid which has between 1000 ppm and 4000 ppm ABA.

In a preferred embodiment, the extract in solid form, is characterised by a DER of between 10:1 and 50:1, preferably by a DER of 10:1 or a DER of 50:1.

In another preferred embodiment, the extract in liquid form as an aqueous solution, is characterised by a DER of 3:1.

In a preferred embodiment, the purified extract also comprises an unpurified extract in a proportion of from 99:1 to 1:99. The unpurified extract is the extract having an ABA content of between 5 ppm and 25 ppm, preferably between 10 ppm and 20 ppm, and which is obtained after extraction from the plant material using the aqueous solvent, according to step a) of the method according to the invention, and optionally after drying the liquid fraction, as described above. Thus an extract may be obtained having an ABA content of between 5 ppm and 4000 ppm, depending on the proportion of each of the extracts. The purified extract and the unpurified extract may be combined in solid and/or liquid form. When both extracts are combined in solid form, the ABA content in said combination of extracts preferably has a value of between 10 ppm and 300 ppm, between 20 ppm and 300 ppm, between 30 ppm and 300 ppm, between 40 ppm and 300 ppm, between 50 ppm and 300 ppm, between 60 ppm and 300 ppm, between 70 ppm and 300 ppm, between 80 ppm and 300 ppm, between 90 ppm and 300 ppm, between 100 ppm and 300 ppm, between 150 ppm and 300 ppm, between 200 ppm and 300 ppm, or between 250 and 300 ppm. When both are combined in liquid form, the ABA content in said combination of extracts preferably has a value of between 20 ppm and 100 ppm, between 30 ppm and 100 ppm, between 40 ppm and 100 ppm, between 50 ppm and 100 ppm, between 60 ppm and 100 ppm, between 70 ppm and 100 ppm, between 80 ppm and 100 ppm, or between 90 ppm and 100 ppm. The combination of one extract in solid form and the other extract in liquid form makes it possible to prepare extracts having different ABA contents. The combination of the purified extract with the unpurified extract makes it possible to prepare extracts having specific ABA concentrations within a wide range of between 5 ppm and 4000 ppm without the need to use additional ingredients and while keeping the plant material matrix used as the initial product for the extraction.

### Compositions

The disclosure also relates to a composition which comprises the extract of the invention and at least one additional ingredient.

The compositions may be pharmaceutical, nutritional or cosmetic.

In an embodiment, the extract is combined with an additional ingredient selected from among the group consisting of colloidal silica, talc, tricalcium phosphate, magnesium stearate, carbohydrates (for example, starch), maltodextrins, water, polyalcohols (for example, glycerine or propylene glycol), celluloses (for example, microcrystalline cellulose), starches and mixtures thereof. In a preferred embodiment, the extract is combined with additional ingredients which have a low glycaemic index, such as, for example, tagatose.

In a preferred embodiment, the composition comprises between 0.5 wt.% and 35 wt.%, preferably between 5 wt.% and 20 wt.% of an additional ingredient. In a more preferred embodiment, it comprises approximately 20 wt.% maltodextrin as an additional ingredient, and in another more preferred embodiment, the composition comprises approximately 5 wt.% silicon dioxide or tricalcium phosphate as an additional ingredient.

The disclosure also relates to the use of said extract in a nutritional, pharmaceutical or cosmetic composition.

The disclosure relates to said extract for use as a medicine. In particular, the extract may be used in the treatment of diseases, as described in the above-mentioned prior art, such as, for example, diabetes, inflammatory bowel disease, atherosclerosis, insulin resistance, hyperglycaemia and glucose intolerance, more preferably in the treatment of diabetes and hyperglycaemia.

### Pharmaceutical compositions

Suitable pharmaceutical compositions for administering the extract and methods for the preparation thereof will be quite clear to persons skilled in the art. Said compositions and methods for the preparation thereof may be found, for example, in Remington The Science and Practice of Pharmacy, 20th edition, ed. A. R. Gennaro, Lippincott Williams and Wilikins, 2000.

Suitable excipients and vehicles for the preparation of said pharmaceutical compositions are known to persons skilled in the art, and may be found in Handbook of Pharmaceutical Excipients, 4th edition, Ed. R. C. Crove, P. J. Sheskey and P. J. Weller, Pharmaceutical Press, 2003.

For suitable administration to a mammal, in particular to a human being, an effective dose of ABA may be given preferably by the oral route (including buccal and sublingual administration), and by the parenteral route (including rectal administration or by injection), although the topical route may also be used (including ocular and nasal administration) and the pulmonary route. The dosage delivery forms include tablets, dispersions, suspensions, solutions, capsules, creams, ointments, aerosols and similar. Preferably, the extract according to the invention is administered by the oral or parenteral route.

As already mentioned, the extract may be administered by the oral route. Oral administration may include swallowing, such that the active ingredient enters the gastrointestinal tract, and/or buccal, lingual or sublingual administration, whereby the active ingredient enters the blood stream directly from the mouth.

Formulations suitable for oral administration include solid, semi-solid and liquid systems such as tablets; soft or hard capsules which contain multiples or nanoparticles, liquids or powders; pills (including those filled with liquid); chewing gums; rapid dispersion dosage delivery forms; films; ovules; aerosols; and buccal/muco-adhesive patches.

Liquid formulations include suspensions, solutions, syrups and elixirs. These types of formulations may be used as fillers in soft or hard capsules (made, for example, of gelatine or hydroxypropyl methylcellulose) and typically comprise a vehicle, for example water, ethanol, propylene glycol, polyethylene glycol, methyl cellulose or a suitable oil, and one or more emulsifying agents and/or suspension agents. Liquid formulations may also be prepared by reconstitution of a solid, for example, from a sachet.

For the tablet dosage delivery form, the tablets usually contain, in addition to the active agent, a disintegrating agent, such as, for example, sodium carboxymethyl cellulose, sodium starch glycolate, calcium carboxymethyl cellulose, croscarmellose sodium, crospovidone, polyvinylpyrrolidone, methyl cellulose, microcrystalline cellulose, lower-alkyl-substituted hydroxypropyl cellulose, starch, pre-gelatinised starch or sodium alginate.

Agglutinating agents are generally used to impart cohesive qualities to a tablet formulation. Suitable agglutinates include, for example, gelatine, microcrystalline cellulose, sugars, polyethylene glycol, natural and synthetic gums, polyvinylpyrrolidone, pre-gelatinised starch, hydroxypropyl cellulose or hydroxypropyl methylcellulose. Tablets may also contain diluents, such as, for example, lactose (monohydrate, spray-dried monohydrate, anhydrous and similar), saccharose, sorbitol, mannitol, xylitol, dextrose, microcrystalline cellulose, starch or calcium phosphate dibasic dihydrate.

Tablets may also optionally comprise surfactants, such as, for example, polysorbate 80, or sodium lauryl sulphate, and sliding agents, such as, for example, silicon dioxide or talc.

Tablets also usually contain lubricants, such as, for example, magnesium stearate, calcium stearate, zinc stearate, sodium stearyl fumarate or mixtures of magnesium stearate with sodium lauryl sulphate.

Other possible ingredients include antioxidants, colorants, flavourings and flavour enhancers, preservatives, saliva stimulating agents, cooling agents, co-solvents (including oils), emollients, bulking agents, anti-foaming agents, surfactants, taste masking agents, or mixtures thereof.

Mixtures of the extract and excipients may be compressed directly or by rollers to form tablets. Said mixtures or a portion thereof may alternatively be wet-granulated, dry-granulated or melt-granulated, melt-frozen, or extruded before tablet formation. The final formulation may comprise one or more layers and may be coated or not coated; it may also be encapsulated.

The formulation of tablets is discussed, for example, in Pharmaceutical Dosage Forms: Tablets. Vol. 1, by H. Lieberman, L. Lachman, J.B. Schwartz (2nd edition, Marcel Dekker, New York, 1989).

Solid formulations for oral administration may be formulated for immediate and/or modified release. Modified release formulations include delayed, sustained, pulsed, controlled, directed and programmed release.

Controlled release compositions for oral use may, for example, be constructed to release the active agent by controlling the dissolution and/or diffusion thereof.

Dissolution or controlled release by diffusion may be achieved by coating a tablet, capsule, granules or granulated active agent formulation appropriately, or by incorporating the active agent in an appropriate matrix. A controlled-release coating may include one or more substances, such as, for example, shellac, beeswax, castor wax, carnauba wax, stearyl alcohol, glyceryl monostearate, glyceryl distearate, glyceryl palmitostearate, ethyl cellulose, dl-polylactic acid, cellulose acetate butyrate, polyvinyl chloride, polyvinyl acetate, vinylpyrrolidone, polyethylene, polymethacrylate, methyl methacrylate, methacrylate hydrogels, 1,3-butylene glycol, ethylene glycol methacrylate and/or polyethylene glycols. In a controlled-release matrix formulation, the matrix material may also include, for example, hydrated methyl cellulose, carnauba wax and stearyl alcohol, Carbopol^{®} 934, silicon, glyceryl tristearate, methyl acrylate-methyl methacrylate, polyvinyl chloride, polyethylene and/or halogenated fluorocarbon.

A controlled-release composition that contains the extract may also be in the form of a floating tablet or capsule (that is, a tablet or capsule which, after oral administration, floats on the gastric contents for a certain period of time). A floating tablet formulation of the active agent or agents may be prepared by granulating a mixture of the extract with excipients and hydrocolloids, such as hydroxyethyl cellulose, hydroxypropyl cellulose or hydroxypropyl methylcellulose. The granules obtained may then be compressed into tablets. In contact with the gastric juices, the tablet forms a gel barrier that is substantially impermeable to the water around its surface. This gel barrier helps to maintain a density of less than one, thus allowing the tablet to remain floating on the gastric juices.

The extract may also be administered by the parenteral route directly into the blood stream, into the muscle or into an internal organ. Suitable means for parenteral administration include intravenous, intraarterial, intraperitoneal, intrathecal, intraventricular, intraurethral, intrasternal, intracraneal, intramuscular, intrasynovial and subcutaneous administration. Suitable devices for parenteral administration include needle injectors (including microneedles), needleless injectors and infusion techniques.

Parenteral formulations are typically aqueous solutions which may contain excipients, such as, for example, salts, carbohydrates and buffering agents (preferably having a pH of 3 to 9), but, for some applications, said formulations may be formulated more appropriately as a sterile, non-aqueous solution or as a dry form to be used in conjunction with a suitable vehicle such as pyrogen-free sterile water.

Preparing parenteral formulations under sterile conditions, for example by lyophilisation, may be carried out easily using standard pharmaceutical techniques that are well known to persons skilled in the art.

Formulations for parenteral administration may be formulated for immediate and/or modified release. Modified-release formulations include delayed, sustained, pulsed, controlled, directed and scheduled release. Thus, the ABA extract may be formulated as a suspension or as a liquid, solid, semisolid or thixotropic for administration as an implanted deposit which provides modified release of the active ingredient. Examples of such formulations include stents coated with active agents, and semisolids and suspensions which comprise microspheres of poly(lactic-co-glycolic acid) (PGLA) loaded with an active agent.

The ABA extract may also be administered topically, (intra) dermically or transdermically to the skin or to the mucosa. Typical formulations for this purpose include gels, hydrogels, lotions, solutions, creams, ointments, powders for sachets, dressings, foams, films, skin patches, wafers, implants, sponges, fibres, bandages and microemulsions. Liposomes may also be used. Typical vehicles include alcohol, water, mineral oil, liquid petroleum jelly, white petroleum jelly, glycerine, polyethylene glycol and propylene glycol.

The ABA extract may also be administered intranasally or by inhalation, typically in the form of a dry powder (either alone, as a mixture, for example, in a dry mixture with lactose, or as a mixed-ingredient particle, for example, mixed with phospholipids, such as phosphatidylcholine) of a dry powder inhaler or as an aerosol spray from a pressurised container, pump, spray, atomiser (preferably an atomiser using electrohydrodynamics to produce a fine mist) or nebuliser, with or without the use of a suitable propellant, such as 1,1,1,2-tetrafluoroethane or 1,1,1,2,3,3,3-heptafluoropropane, or as nasal drops. For intranasal use, the powder may comprise a bioadhesive agent, for example, chitosan or cyclodextrin.

The pressurised container, pump, spray, atomiser or nebuliser contains the ABA extract, a suitable solvent and, possibly, a surfactant.

Before use in a dry powder formulation or suspension, the ABA extract is dried and ground. This may be achieved by any suitable trituration method, such as spiral jet milling, fluidised bed jet milling, supercritical fluid processing to form nanoparticles, high-pressure homogenisation or spray drying.

Capsules (made, for example, of gelatine or hydroxypropyl methylcellulose), ampoules and cartridges for use in an inhaler or puffer may be formulated to contain a powdered mixture of the ABA extract, a suitable powder base such as lactose or starch, and a performance modifier such as leucine, mannitol or magnesium stearate. Lactose may be anhydrous or in monohydrate form, preferably the latter. Other suitable excipients include dextran, glucose, maltose, sorbitol, xylitol, fructose, saccharose and trehalose.

Formulations intended for administration by inhalation/intranasally may be formulated to be immediate release and/or modified release using, for example, PGLA.

The ABA extract may also be administered rectally or vaginally, for example, in the form of a suppository, a pessary or an enema. Cocoa butter is a conventional suppository base, but various alternatives may be used as appropriate.

### Nutritional compositions

The ABA extract may form part of a nutritional composition, such as, for example, nutritional supplements. In these types of supplements, the ABA may be present in doses that are suitable for daily (or more frequently) administration. Typically, for dietetic supplements, the ABA is present in a form suitable for oral ingestion, such as by means of pills, capsules, tablets, powders, liquids or similar. As with pharmaceutical compositions, typical additives may be included, such as disintegrating agents, agglutinating agents, gums, colourants, or flavourings.

### Cosmetic compositions

Cosmetic compositions which comprise the ABA extract normally include an additional ingredient selected from the group which comprises, for example, surfactants, emulsifiers, lipid compounds, emollients, consistency factors, thickening agents, hydrotropes, and preservatives
The compositions may include surfactants to facilitate the solution, emulsion, dispersion or suspension of the cosmetic ingredients.

Surfactants may be anionic, non-ionic, cationic and/or amphoteric. Preferably, non-ionic surfactants are used. Among the anionic surfactants, the following may be cited for example: soaps, sulphonated alkanes, sulphonated olefins, alkyl sulphates, fatty alcohol ether sulphates, glycerine ether sulphates, fatty acid ether sulphates, mono- and di-alkyl sulphosuccinates, mono- and di-alkyl sulphosuccinamates, ether carboxylic acids and the salts thereof, fatty acid isethionates, fatty acid sarcosinates, fatty acid taurides, N-acyl amino acids. Among the non-ionic surfactants, the following may be cited for example: polyalkoxylated fatty alcohols, polyalkoxylated fatty acids, polyalkoxylated fatty acid amides, polyalkoxylated fatty amines, alkoxylated triglycerides, mixed ethers, alkyl polyglycosides, N-alkyl fatty acid glucamides, sorbitan esters, polyethoxylated sorbitan esters, and amine oxides. Among the cationic surfactants, the following may be cited for example: quaternary ammonium compounds, and quaternary salts of trialkanolamine esters and fatty acids, for example esterquats. Among the amphoteric surfactants, the following may be cited for example: alkyl betaines, alkyl amido betaines, amino propionates, amino glycinates, imidazoline betaines and sulphobetaines.

The cosmetic compositions usually contain a series of additional lipid compounds and emollients, which help optimise the organoleptic and dermatological properties thereof. The following are possible lipid compounds: fatty alcohol-based Guerbet alcohols having 6 to 18 carbon atoms, linear fatty acid esters having 6 to 22 carbon atoms containing linear alcohols having 6 to 22 carbon atoms, branched carboxylic acid esters having 6 to 13 carbon atoms containing linear alcohols having 6 to 22 carbon atoms, such as, for example, myristyl myristate, myristyl palmitate, myristyl stearate, behenyl stearate, behenyl isostearate, behenyl oleate, behenyl behenate, behenyl erucate, or erucyl myristate. The following are also suitable: linear fatty acid esters having 6 to 22 carbon atoms containing branched alcohols; alkyl hydroxy carboxylic acids esters having 18 to 38 carbon atoms containing linear or branched fatty alcohols having 6 to 22 carbon atoms, in particular dioctyl malates; linear and/or branched fatty acid esters containing polyvalent alcohols, and/or Guerbet alcohols; fatty acid-based triglycerides having 6 to 10 carbon atoms; liquid mixtures of fatty acid-based mono-/di-/triglycerides having 6 to 18 carbon atoms; fatty alcohol esters having 6 to 22 carbon atoms, and/or Guerbet alcohols having aromatic carboxylic acids, in particular benzoic acid; dicarboxylic acid esters having 2 to 12 carbon atoms containing linear or branched alcohols having 1 to 22 carbon atoms, or polyols having 2 to 10 carbon atoms and 2 to 6 hydroxyl groups; vegetable oils; branched primary alcohols; substituted cyclohexanes; linear or branched fatty alcohol carbonates having 6 to 22 carbon atoms, such as, for example, dicaprylyl carbonates; linear or branched, symmetrical or asymmetrical dialkyl ethers having 6 to 22 carbon atoms per alkyl group, such as, for example, dicaprylyl ethers; aliphatic hydrocarbons, or alternatively naphthenics, such as squalane, squalene or dialkyl cyclohexanes, and mixtures thereof.

Consistency factors and thickeners are used in the cosmetic compositions to adjust the viscosity and the rheological behaviour thereof. First of all, possible consistency factors that may be considered are fatty alcohols having a chain of 12 to 22 carbon atoms, and preferably of 16 to 18 carbon atoms, and in addition partial glycerides, fatty acids or hydroxylated fatty acids. Suitable thickening agents are, by way of example, anhydrous hydrophilic silicon; polysaccharides, especially xanthan gum, guar gum, agar-agar, alginates and thyloses, carboxymethyl cellulose and hydroxyethyl cellulose, as well as polyethylene glycol mono- and diesters of fatty acids of a higher molecular weight; polyacrylates, polyacrylamides, polyvinyl alcohol and polyvinylpyrrolidone.

To improve the fluidity of the cosmetic compositions, hydrotropes may also be used, such as, for example, ethanol, isopropyl alcohol or polyols. The polyols considered in the case preferably have 2 to 15 carbon atoms, and at least two hydroxyl groups. The polyols may also contain other functional groups, in particular, amino groups, or alternatively may be modified with nitrogen. Typical examples are glycerine; alkylene glycols, such as ethylene glycol, diethylene glycol, propylene glycol, butylene glycol, hexylene glycol, as well as polyethylene glycols of an average molecular weight of 100 to 1000 daltons; technical mixtures of oligo glycerines having a typical condensation level of 1.5 to 10, such as, for example, technical mixtures of diglycerines having a diglycerine content of 40 to 50 wt.%; methylol compounds, such as, in particular, trimethylol methane, trimethylol propane, trimethylol butane, pentaerythritol and dipentaerythritol; alkyl glucosides having a chain of 1 to 8 carbon atoms in the alkyl residue; saccharic alcohols having 5 to 12 carbon atoms, such as, for example, sorbitol or mannitol; sugars having 5 to 12 carbon atoms, such as, for example, glucose or saccharose; amino sugars, such as, for example, glucamine; dialcoholamines, such as diethanolamine or 2-amino-1,3-propanediol.

Suitable preservative agents for use in the compositions are, by way of example, phenoxyethanol, formaldehyde solution, parabens (methylparaben, propylparaben, butylparaben and mixtures thereof), 3-(4-chlorophenoxy)-1,2-propanediol (chlorphenesin), pentanediol or sorbic acid, and the additional substance classes indicated in Annex VI to the Cosmetics Directive 76/768/EEC.

The present invention discloses a method for preparing a botanical extract of abscisic acid from dried figs, characterised in that it comprises:
a) extracting a plant material which contains abscisic acid using an aqueous solvent, and separating the solid plant material from the liquid fraction,
b) concentrating the liquid fraction,
c) purifying the liquid fraction obtained at step b) by chromatographic separation in an adsorption column using an organic solvent miscible in water as an eluent, or a liquid-liquid extraction using a solvent that is not miscible in water, and
d) evaporating the organic phase obtained at step c);

wherein in step a) said aqueous solvent is water at a temperature of between 50 ºC to 75ºC; and
wherein said botanical extract of abscisic acid comprises between 1000 ppm and 4000 ppm abscisic acid.

Said plant material which contains abscisic acid is selected from dried figs.

Said plant material is dry.

In said method, the aqueous solvent is selected from water.

In said method, extraction is carried out using water as the only solvent.

Using water alone as extraction solvent is an option wherein said solvent is safe and toxicity is zero.

At industrial scale, is preferably to use percolation as extraction step. Said percolation is made in industrial equipment which can operate equi-current or countercurrent. The flow rate of extraction solvent in said percolation step is within a range of 500 to 1200 liters per hour.

The amount of raw plant material loaded in each industrial batch is between 500 and 2000 kg, depending on the industrial equipment used. The amount of the resulting extract (extraction micelle) is about 1200 liters.

In said method, a volume of solvent is used which is equivalent to between 3 and 6 times the weight of the plant material to be extracted. With said range of ratio of volume of solvent per weight of plant material it is possible to establish a balance between process efficiency and cost-effectiveness thereof at industrial scale.

The extraction step of the method of the present invention a recovery of more than 75% of the starting content of ABA, making said process cost-effective from the industrial point of view. Taking into account that content of ABA in starting plant material is very low, it is very important to make an adequate selection of the raw material with the aim of achieving a cost-effective extraction at industrial scale.

One of the plant raw material having a higher content of ABA are figs or citric fruits. In industrializing a process of extracting figs is necessary to start with a raw material containing more than 1 ppm of ABA, preferably more than 5 ppm. Said selection involves a rigurous analysis of the raw material, comprising from a representative sampling to applying appropriate analytical techniques.

Also at industrial scale, where a large volume of extract is obtained and sugar concentration in said extract is high, it makes said extract very sensitive to microbial degradation. To avoid this, it is preferably to pasteurize the extract obtained in order to keep its microbiological integrity during the concentration and purification steps.

Extraction in said method is carried out at a temperature of between 50ºC and 75ºC.

In the process of the present invention it is preferable to pasteurize said extract at least at 92ºC during at least 78 seconds, more preferable at 98ºC during 90 seconds.

Purification in said method is carried out by chromatographic separation in an adsorption column using an organic solvent miscible in water as an eluent.

The organic solvent miscible in water used in said adsorption column is selected from the group consisting of a lower aliphatic alcohol having 1 to 4 carbon atoms, ketone, dioxane, tetrahydrofuran, acetonitrile and mixtures thereof. Said organic solvent miscible in water is a lower aliphatic alcohol having 1 to 4 carbon atoms.

The eluent of said method is selected from the group consisting of methanol and ethanol. More preferably, the eluent is ethanol.

In the liquid-liquid extraction of said method, an organic solvent that is not miscible in water is used, selected from among the group consisting of butanol and ethyl acetate. Said solvent is ethyl acetate.

Disclosed is is a botanical extract of abscisic acid obtained according to said method, characterised in that it comprises between 5 ppm and 4000 ppm abscisic acid.

Said botanical extract of abscisic acid comprises between 1000 ppm and 4000 ppm abscisic acid.

Moreover, the extract comprises an unpurified extract in a proportion of 99:1 to 1:99, in which the unpurified extract corresponds to the extract obtained after extraction from the plant material using the aqueous solvent, according to step a) of the method of the present invention, and, in any case, after drying of the liquid fraction.

Said extract comprises between 5 ppm and 4000 ppm abscisic acid.

A composition is disclosed which comprises said extract and, at least, one additional ingredient.

In said composition, the additional ingredient is selected from among the group consisting of colloidal silica, talc, tricalcium phosphate, magnesium stearate, carbohydrates, maltodextrins, water, polyalcohols, celluloses, starches and mixtures thereof.

Said composition comprises between 0.5 wt.% and 35 wt.% of an additional ingredient. More preferably, said composition comprises 20 wt.% maltodextrin. Said composition comprises 5 wt.% silicon dioxide or tricalcium phosphate.

In another embodiment, the use of the extract in a nutritional, pharmaceutical or cosmetic composition is disclosed.

Furthermore, the use of said extract as a medicine is disclosed.

Various examples are given below as non-limiting illustrative examples of the invention.

### Examples

ABA can be determined by methods that are well known to a person skilled in the art, which include, generally, HPLC, mass spectrometry, or more conventional detectors, such as, for example, UV spectrophotometers, as described for example in the article by Kelen et al., Separation of Abscisic Acid, Indole-3-Acetic Acid, Gibberellic Acid in 99 R (Vitis berlandieri x Vitis rupestris) and Rose Oil (Rosa damascena Mill.) by Reversed Phase Liquid Chromatography, Turk J. Chem, 2004, 29, 603-610.

### Example 1: Preparation of an ABA extract using water (does not form part of the invention)

50 g of dried figs cut into chunks with a size of less than 2 cm were extracted using warm water at a temperature of between 55 and 65°C, while stirring. Two extraction steps were carried out, based on adding a quantity of solvent equivalent to between 3 and 6 times the weight of the plant material to be extracted.

The plant material was separated from the resulting micelle by solid-liquid filtration. The micelle was concentrated by vacuum evaporation, while maintaining the temperature below 65°C, until a product was obtained which had a dry residue of 78 wt.%. The product was vacuum dried and at a temperature of no more than 70ºC. Thus, 25 g of dry extract were obtained, which corresponds to an extraction yield of 50%, that is, a DER of 2:1.

The ABA content in this extract was 19 ppm.

Various extraction operations from dried figs using warm water led to a dry extract being obtained with an ABA content of between 5 and 25 ppm.

### Example 2: Preparation of an ABA extract using a mixture of water and ethanol (does not form part of the invention)

50.5 g of dried figs cut into chunks with a size of less than 2 cm were extracted using 500 ml of a mixture of ethanol and water having an ethanol content of 70 vol.%. Extraction was carried out at a temperature of between 55 and 65°C, while stirring and for 2 hours.

The plant material was separated from the micelle by solid-liquid filtration. The micelle was concentrated by vacuum evaporation, while maintaining the temperature below 65°C, until a product was obtained which had a dry residue of 76 wt.%. The product was vacuum dried and at a temperature of no more than 70ºC. Thus, 26.3 g of dry extract were obtained, which corresponds to a DER of 1.92:1. The ABA content in this extract was 10 ppm.

### Example 3: Preparation of an ABA extract using a mixture of water and ethanol (does not form part of the invention)

20.4 g of dried orange peel were extracted using 2 x 200 ml of a 70 vol.% mixture of ethanol and water for an hour while stirring and at a temperature of 50ºC.

Following a method that was substantially similar to that described in example 2, 7.4 g of an extract were obtained (DER 2.76:1), which had an ABA content of 6 ppm.

### Example 4: Preparation of an ABA extract using a mixture of water and ethanol (does not form part of the invention)

20.7 g of dried mandarin peel were extracted using 2 x 200 ml of a 70 vol.% mixture of ethanol and water for an hour while stirring and at a temperature of 50ºC.

Following a method that was substantially similar to that described in example 2, 8.4 g of an extract were obtained (DER 2.46:1), which had an ABA content of 8 ppm.

### Example 5: Preparation of an ABA extract using a mixture of water and ethanol (does not form part of the invention)

50.1 g of dried bitter orange peel were extracted in three steps using 350 ml, 250 ml and 200 ml of a 70 vol.% mixture of ethanol and water for an hour while stirring and a temperature of 50ºC.

Following a method that was substantially similar to that described in example 2, 17.3 g of an extract were obtained (DER 2,9:1), which had an ABA content of 16 ppm.

### Example 6: Preparation of an ABA extract using a mixture of water and ethanol (does not form part of the invention)

50.3 g of small, whole, dried bitter oranges were extracted in three steps using 250 ml, 250 ml and 150 ml of a 70 vol.% mixture of ethanol and water for an hour while stirring and a temperature of 50ºC.

Following a method that was substantially similar to that described in example 2, 19.4 g of an extract were obtained (DER 2.59:1), which had an ABA content of 20 ppm.

### Example 7: Preparation of a purified extract of ABA

500 g of whole dried figs underwent two extraction steps. Each of the steps lasted for two hours and 2500 ml of water were used at a temperature of 70ºC.

The plant material was separated from the micelle by solid-liquid filtration. The micelle had a weight of 4636 g and a dry residue of 5.5 wt.%. Said micelle was concentrated by vacuum evaporation, while maintaining the temperature below 65°C, until a product was obtained which had a dry residue of 10 wt.%.

This concentrated product was loaded into a chromatographic column filled with an adsorption resin.

Elution of the products contained in the column was carried out using ethanol as the eluent. The ethanolic solution was concentrated until a dry residue of 75 wt.% was obtained, and drying was continued in a vacuum oven and at a temperature of no more than 65ºC.

Finally, 2.1 g of purified ABA extract were obtained, meaning a yield of 0.42% on the initial raw material, that is, a DER of 240:1. The ABA content is 1400 ppm.

### Example 8: Preparation of a purified ABA extract

100 kg of whole dried figs were loaded in four 30 l percolators and extraction was carried out using warm water at a temperature of 70ºC, in a proportion of approximately six times by weight with respect to the mass of the plant material to be extracted.

The micelle, 1050 I, had a dry residue of 4.9 wt.%, and was concentrated by vacuum evaporation, while maintaining the temperature below 65°C, until a product was obtained that had a dry residue of 10 wt.%.

Next, two extractions were carried out using 1000 l of ethyl acetate, each one having three times the volume and organic phase compared with the aqueous phase, while stirring and at a temperature of 50ºC.

The combined organic fractions were concentrated until a dry residue of 75 wt.% was obtained, and drying was continued in a vacuum oven and at a temperature of no more than 65ºC.

120 g of an extract were obtained having an ABA content of 3800 ppm. The yield by this purification method is 0.1-0.12 wt.%, which corresponds to an approximate DER of 850-1000:1.

### Example 9: Nutritional composition containing ABA extract (does not form part of the invention)

12 kg of whole dried figs were loaded in a 30 l percolator and extraction was carried out using warm water at a temperature of 70ºC, in a proportion of approximately 11 times the weight of the plant material.

The micelle had a dry residue of 4.5 wt.%, and was concentrated by vacuum evaporation, while maintaining the temperature below 65°C, until a product was obtained which had a dry residue of 79 wt.%.

Tapioca maltodextrin was added to said product in a proportion of 20 wt.% of the final dry material, and drying of the composition was completed in an oven, while maintaining a temperature of no more than 70°C. 7.5 kg of crude extract were obtained having an ABA content of 10 ppm.

Similar compositions were prepared using 5 wt.% of anhydrous silicon and 5 wt.% of tricalcium phosphate instead of maltodextrin.

### Example 10: Preparation of yogurt containing ABA extract (does not form part of the invention)

1 l of pasteurised cows' milk was mixed with lactic starters and said mixture was heated to a temperature of 45ºC. The mixture was maintained at said temperature for 8 hours to promote the fermentation thereof. At the end of this period, the mixture was cooled quickly to 4ºC. 50 g of sugar were then added and 0.26 g of an extract having an ABA content of 300 ppm. A pH regulating agent was added to ensure the viability of the lactic starters.

### Example 11: Preparation of cereal bars containing ABA extract (does not form part of the invention)

Oatmeal, dates, cranberries/blueberries, sesame seeds and almonds were ground. Mixing was carried out until a homogenous composition was achieved. Sugar and 1 g of an extract having an ABA content of 50 ppm were added to the mixture. The paste was placed on baking paper and stretched to a thickness of 0.5 cm. It is left to rest in the refrigerator for at least 2 hours.

### Example 12: Preparation of an energy drink containing ABA extract (does not form part of the invention)

100 ml of squeezed lemon juice were added to 60 ml of natural honey and mixed with 250 ml of water. 2 g of salt, 2 g bicarbonate of soda and 4 g of an extract having an ABA content of 20 ppm were added. The mixture was stirred until the ingredients had completely dissolved. It was left to cool in a covered container.

### Example 13: Preparation of body cream containing ABA extract (does not form part of the invention)

200 ml of a commercial cream were mixed with 0.2 g of an extract having an ABA content of 500 ppm and 10 ml of coconut oil.

### Example 14: Preparation of tablets containing ABA extract (does not form part of the invention)

The excipients are mixed with 0.1 g of an extract having an ABA content of 300 ppm. The mixture was agglomerated. Three tablets a day should be taken in order to ingest 90 micrograms of ABA daily.

### Example 15: Preparation of sachets containing ABA extract (does not form part of the invention)

The excipients are mixed with 0.23 g of an extract having an ABA content of 300 ppm. It was wrapped in waterproof paper sachets. The content of a sachet should be dissolved in water for oral ingestion.

### Example 16: Preparation of capsules containing ABA extract (does not form part of the invention)

0.15 g of an extract having an ABA content of 300 ppm were introduced in a hard gelatine capsule. Two capsules a day should be consumed in order to ingest 90 micrograms of ABA daily.

### Example 17: Preparation of a purified extract of ABA using filtration (does not form part of the invention)

10 kg of whole dry figs were extracted using 100 kg of water at 70ºC with two extraction steps. The plant material was separated from micelles by a solid liquid filtration resulting in a liquid with a concentration of solids of about 8.6% and an ABA content of 10 ppm. Said micelle was subjected to a tangential flow filtration using a filtration cartridge with a nanofiltration membrane. For this process, the micelle pH was adjusted between 2 and 5 by adding a mineral acid. The pressure of the filtration process was set between 4 and 20 bar.

After filtration, about 80% of the starting ABA was recovered versus about 20% obtained in the permeate, resulting in a purification of three times of the starting ABA content.

### Example 18: Preparation of a purified extract of ABA using filtration (does not form part of the invention)

8 kg of whole dry figs were extracted with 100 kg of water at 70ºC with two extraction steps. The plant material was separated from micelles by a solid liquid filtration resulting in a liquid with a concentration of solids of about 8.6% and an ABA content of 9.1 ppm. Said micelle was subjected to a tangential flow filtration using a filtration cartridge with a nanofiltration membrane. For this process, the micelle pH was adjusted between 5 and 9 by adding a mineral acid. The pressure of the filtration process was set between 4 and 20 bar.

After filtration, about 95% of the starting ABA was recovered versus about 5% obtained in the permeate, resulting in a purification of more than 5 times of the starting ABA content.

### Example 19: Preparation of a purified extract of ABA using filtration (does not form part of the invention)

5 kg of whole dry figs were extracted with 100 kg of water at 70ºC with two extraction steps. The plant material was separated from micelles by a solid liquid filtration resulting in a liquid with a concentration of solids of about 8.0% and an ABA content of 10 ppm. Said micelle was subjected to a tangential flow filtration using a filtration cartridge with a nanofiltration membrane with a pore size of > 1000 Da. For this process, the micelle pH was adjusted between 5 and 9 by adding a mineral acid. The pressure of the filtration process was set between 4 and 20 bar.

After filtration, about 65% of the starting ABA was recovered, resulting in a purification of 2 times of the starting ABA content.

### Example 20: Preparation of a purified extract of ABA using filtration (does not form part of the invention)

8 kg of whole dry figs were extracted with 100 kg of water at 70ºC with two extraction steps. The plant material was separated from micelles by a solid liquid filtration resulting in a liquid with a concentration of solids of about 9.0% and an ABA content of 8.9 ppm. Said micelle was subjected to a tangential flow filtration using a filtration cartridge with a nanofiltration membrane with a pore size of > 1000 Da. For this process, the micelle pH was adjusted between 2 and 5 by adding a mineral acid. The pressure of the filtration process was set between 4 and 20 bar.

After filtration, about 60% of the starting ABA was recovered, resulting in a purification of 2 times of the starting ABA content.

## Claims

1. Method for preparing a botanical extract of abscisic acid from dried figs, comprising:
a) extracting a plant material which contains abscisic acid using an aqueous solvent, and separating the solid plant material from the liquid fraction,
b) concentrating the liquid fraction,
c) purifying the liquid fraction obtained at step b) by chromatographic separation in an adsorption column using an organic solvent miscible in water as an eluent, or liquid-liquid extraction using a solvent that is not miscible in water, and
d) evaporating the organic phase obtained at step c);
wherein in step a) said aqueous solvent is water at a temperature of between 50 °C to 75°C; and
wherein said botanical extract of abscisic acid comprises between 1000 ppm and 4000 ppm abscisic acid.

2. Method according to claim 1, **characterised in that** a volume of water is used that is equivalent to between 3 and 6 times the weight of the dried figs to be extracted.

3. Method according to any one of claims 1 to 2, **characterised in that** purification is carried out by chromatographic separation in an adsorption column using an organic solvent miscible in water as an eluent.

4. Method according to claim 3, **characterised in that** the eluent is ethanol.

5. Method according to any one of claims 1 to 4, **characterised in that** in the liquid-liquid extraction the organic solvent used is ethyl acetate.

6. Method, according to claim 1 to 5, **characterised in that** said extraction step a) is made by percolation.

## Patentansprüche

1. Verfahren zur Herstellung eines pflanzlichen Abscisinsäureextrakts aus getrockneten Feigen, umfassend:
a) Extrahieren eines Abscisinsäure enthaltenden Pflanzenmaterials unter Verwendung eines wässrigen Lösungsmittels und Abtrennen des festen Pflanzenmaterials von der flüssigen Fraktion,
b) Konzentrieren der flüssigen Fraktion,
c) Reinigen der in Schritt b) erhaltenen flüssigen Fraktion durch chromatographische Trennung in einer Adsorptionssäule unter Verwendung eines mit Wasser mischbaren organischen Lösungsmittels als Elutionsmittel oder durch Flüssig-Flüssig-Extraktion unter Verwendung eines mit Wasser nicht mischbaren Lösungsmittels, und
d) das Eindampfen der in Schritt c) erhaltenen organischen Phase;
wobei in Schritt a) das wässrige Lösungsmittel Wasser mit einer Temperatur zwischen 50 °C und 75 °C ist; und
wobei der pflanzliche Abscisinsäureextrakt zwischen 1000 ppm und 4000 ppm Abscisinsäure enthält.

2. Verfahren nach Anspruch 1, **dadurch gekennzeichnet, dass** ein Wasservolumen verwendet wird, das dem 3- bis 6-fachen des Gewichts der zu extrahierenden getrockneten Feigen entspricht.

3. Verfahren nach einem der Ansprüche 1 bis 2, **dadurch gekennzeichnet, dass** die Reinigung durch chromatographische Trennung in einer Adsorptionssäule unter Verwendung eines mit Wasser mischbaren organischen Lösungsmittels als Elutionsmittel durchgeführt wird.

4. Verfahren nach Anspruch 3, **dadurch gekennzeichnet, dass** das Elutionsmittel Ethanol ist.

5. Verfahren nach einem der Ansprüche 1 bis 4, **dadurch gekennzeichnet, dass** bei der Flüssig-Flüssig-Extraktion das verwendete organische Lösungsmittel Ethylacetat ist.

6. Verfahren nach einem der Ansprüche 1 bis 5, **dadurch gekennzeichnet, dass** der Extraktionsschritt a) durch Perkolation durchgeführt wird.

## Revendications

1. Procédé de préparation d'un extrait botanique d'acide abscissique à partir de figues séchées, comprenant les étapes consistant à :
a) extraire une matière végétale contenant de l'acide abscissique à l'aide d'un solvant aqueux, et séparer la matière végétale solide de la fraction liquide,
b) concentrer la fraction liquide,
c) purifier la fraction liquide obtenue à l'étape b) par séparation chromatographique dans une colonne d'adsorption en utilisant un solvant organique miscible à l'eau comme éluant, ou par extraction liquide-liquide en utilisant un solvant non miscible à l'eau, et
d) évaporer la phase organique obtenue à l'étape c) ;
dans lequel, à l'étape a), ledit solvant aqueux est de l'eau à une température comprise entre 50 °C et 75 °C ; et
dans lequel ledit extrait botanique d'acide abscissique comprend entre 1 000 ppm et 4000 ppm d'acide abscissique.

2. Procédé selon la revendication 1, **caractérisé en ce qu'**on utilise un volume d'eau équivalent à 3 à 6 fois le poids des figues séchées à extraire.

3. Procédé selon l'une quelconque des revendications 1 à 2, **caractérisé en ce que** la purification est effectuée par séparation chromatographique dans une colonne d'adsorption en utilisant un solvant organique miscible à l'eau comme éluant.

4. Procédé selon la revendication 3, **caractérisé en ce que** l'éluant est de l'éthanol.

5. Procédé selon l'une quelconque des revendications 1 à 4, **caractérisé en ce que**, dans l'extraction liquide-liquide, le solvant organique utilisé est l'acétate d'éthyle.

6. Procédé selon l'une quelconque des revendications 1 à 5, **caractérisé en ce que** ladite étape d'extraction a) est réalisée par percolation.
